# EUROPÄISCHE PATENTSCHRIFT

(11) **EP 4 192 542 B1**
(45) Veröffentlichungstag und Bekanntmachung des Hinweises auf die Patenterteilung: **24.01.2024**
(21) Anmeldenummer: 22728632.5
(22) Anmeldetag: 23.05.2022
(51) Int. Cl.: A61M 1/36, B01D 63/06, B01D 69/04, B01D 69/12, B01D 71/48, B01D 71/56

(54) **FILTERELEMENT UND VERFAHREN ZU SEINER HERSTELLUNG**
BLOOD FILTER ELEMENT AND METHOD FOR THE PRODUCTION OF SAME
ÉLÉMENT FILTRE À SANG ET SON PROCÉDÉ DE FABRICATION

(30) Priorität: 02.06.2021 EP 21177303
(43) Veröffentlichungstag der Anmeldung: 14.06.2023
(73) Patentinhaber: Sefar AG, 9410 Heiden (CH)
(72) Erfinder: DÜR, Hansjörg, 6858 Schwarzach (AT)
(74) Vertreter: Wunderlich & Heim Patentanwälte Partnerschaftsgesellschaft mbB
(86) Internationale Anmeldenummer: PCT/EP2022/063896
(87) Internationale Veröffentlichungsnummer: WO 2022/253613

(56) Entgegenhaltungen:
- GB-A- 2 222 962
- US-A- 5 192 439

## Beschreibung

Die Erfindung betrifft ein Filterelement, insbesondere ein Blutfilterelement, welches taschen- oder schlauchförmig mit einem sich in einer Längsrichtung erstreckenden Innenraum ausgebildet ist und einen mehrlagigen Aufbau aufweist, welcher eine durchlässige Außenlage, eine durchlässige Innenlage und mindestens eine zwischen der Außenlage und der Innenlage angeordnete Zwischenlage aufweist, welche als ein feinporiges Filterelement ausgebildet ist, wobei das Filterelement eine vorgegebene axiale Länge aufweist, und wobei die Außenlage und die Innenlage die vorgegebene axiale Länge aufweisen, gemäß dem Oberbegriff des Anspruchs 1.

Die Erfindung betrifft weiterhin ein Verfahren zum Herstellen eines solchen Filterelement, insbesondere eines solchen Blutfilterelementes, gemäß dem Anspruch 13. Derartige Filterelement, die als Blutfilter verwendet werden, werden insbesondere bei sogenannten Herz-Lungen-Maschinen zum Reinigen von zirkulierendem Blut und zum Rückhalten zu großer Gasbläschen im Blut eingesetzt.

Aus der WO 98/15302 A1 ist ein Blutfilterelement mit drei Lagen bekannt, wobei die einzelnen Lagen an einem starren Grundkörper oder Rahmen angebracht sind. Durch den Rahmen wird eine zylindrische Form vorgegeben. Ein ähnliches Filterelement ist aus US 5,192,439 bekannt.

Aus der DE 2 530 413 C3 geht ein beutelförmiger Blutfilter hervor, welcher durch Verschweißen verschiedener Lagen gebildet ist.

Der Erfindung liegt die **Aufgabe** zugrunde, ein einfaches und robust aufgebautes Filterelement, insbesondere ein Blutfilterelement, und ein Verfahren zu seiner Herstellung anzugeben, mit welchen eine besonders effiziente Filtrierung, insbesondere von Blut, durchgeführt werden kann.

Die Aufgabe wird zum einen durch ein Filterelement mit den Merkmalen des Anspruchs 1 und zum anderen durch ein Verfahren mit den Merkmalen des Anspruchs 13 gelöst. Bevorzugte Ausführungsformen der Erfindung sind in den abhängigen Ansprüchen angegeben.

Das erfindungsgemäße Filterelement ist dadurch gekennzeichnet, dass die Zwischenlage axial kurzer als die Außenlage und die Innenlage unter Ausbildung eines Überlaufbereiches ausgebildet ist, welcher in einem axialen oberen Endbereich des Filterelementes angeordnet ist, wobei eine Oberkante der Zwischenlage von den Oberkanten der Außenlage und der Innenlage axial nach unten beabstandet ist, dass die Außenlage, die Innenlage und die Zwischenlage entlang mindestens einem Längsrand unter Ausbildung eines Längsverbindungssaumes fest miteinander verbunden sind, und dass die Außenlage und die Innenlage entlang ihrer Oberkante unter Ausbildung eines oberen Querverbindungssaumes fest miteinander verbunden sind, wobei die Oberkante der Zwischenlage frei und unverbunden zwischen der Außenlage und der Innenlage angeordnet ist.

Eine Grundidee der Erfindung liegt darin, dass bei einem mehrlagigen Aufbau eines socken- oder schlauchförmigen Filterelementes das Filterelement als eine Zwischenlage zwischen einer Außenlage und einer Innenlage, jedoch mit einer unterschiedlichen Länge hierzu, ausgebildet wird. Die Zwischenlage wird dabei definiert axial kürzer ausgeführt, so dass eine Oberkante der Zwischenlage unter Ausbildung eines Freiraumes von den Oberkanten der Außenlage und der Innenlage beabstandet ist. In dem oberen Abschnitt des Filterelementes ist somit ein nur zweilagiger Aufbau gegeben, wodurch ein Überlaufbereich gebildet ist. Der Überlaufbereich ohne die Zwischenlage weist einen geringeren Durchflusswiderstand auf. So kann in bestimmten Situationen überschüssiges Fluid schnell und effizient über den Überlaufbereich abgeführt werden, wobei durch die Außenlage und die Innenlage eine Mindestfilterwirkung sichergestellt ist.

Ein weiterer Aspekt der Erfindung besteht darin, dass es für eine Lagefixierung der Zwischenlage in dem Filterelement ausreichend ist, wenn eine feste Verbindung mit der Außenlage und der Innenlage zumindest entlang einer Längskante mit einem Längsverbindungssaum besteht. Insbesondere beruht die Erfindung auf der Erkenntnis, dass eine Oberkante der Zwischenlage frei und unverbunden bleiben kann. Insbesondere kann so bei der Herstellung des Filterelementes auf den Einsatz zusätzlicher Schmelzfolien zur Verbindung innenliegender Lagen verzichtet werden. Durch ein Verbinden der Zwischenlage entlang einer Längsrandes mit den beiden anderen Lagen und einer Verbindung zwischen der Außenlage und der Innenlage an der Oberkante wird ein hinreichend robuster Aufbau erreicht. Durch eine geringe Anzahl von Verbindungssäumen wird eine hohe und wirksame Filterfläche erreicht.

Somit kann bei einer effizienten Fertigung ein besonders vorteilhafter und funktionsfähiger Aufbau eines Filterelementes erzielt werden.

Das erfindungsgemäße Filterelement wird grundsätzlich so eingebaut, dass eine Anströmung des zu reinigenden Fluides, insbesondere Blutes, in den Innenraum von oben oder vorne erfolgt. Zum Reinigen tritt das Fluid radial durch die etwa hohlzylindrische Umfangswand des Filterelementes nach außen. Das Filterelement kann dabei schlauchförmig mit einer oberen und einer unteren Öffnung versehen sein oder alternativ eine Taschen- oder Sockenform aufweisen, bei welcher eine untere Öffnung verschlossen ist. Das Filterelement wird dabei insgesamt so betrieben, dass in einem normalen Filterbetrieb das zu reinigende Fluid, insbesondere Blut, durch den unteren und mittleren Bereich, welcher dreilagig ausgebildet ist, durch die Wandung des Filterelementes hindurchtritt. Durch eine entsprechende Ausbildung der inneren Lage kann eine zusätzliche Filterwirkung, insbesondere eine Kaskadenfiltration zusammen mit der Zwischenlage erzielt werden. Tritt ein unerwartetes Zusetzen oder eine Schaumbildung im Fluid in diesem Bereich auf, kann ein Übertritt des Fluides auch über den zweilagigen Überlaufbereich radial nach außen erfolgen. Hierdurch wird insbesondere bei Verwendung des Filterelementes als Blutfilterelement ein ausreichender Blutkreislauf, etwa durch eine Herz-Lungen-Maschine und damit für den Patienten sichergestellt.

Grundsätzlich kann das zu reinigende Fluid, wie beispielsweise Blut, auch bei einer alternativen Ausgestaltung radial von außen nach innen fließen.

Eine bevorzugte Ausführungsform der Erfindung besteht darin, dass die Außenlage, die Innenlage und/oder die Zwischenlage an ihrer Unterkante unter Ausbildung eines unteren Querverbindungssaumes fest miteinander verbunden sind. Dabei können jeweils nur zwei Lagen oder alle Lagen ringförmig miteinander verbunden sein. Hierdurch wird die Stabilität des Aufbaus erhöht. Für ein taschenförmiges Filterelement ist der untere Querverbindungssaum so ausgebildet, dass eine Öffnung des Innenraumes nach unten vollständig verschlossen ist.

Allgemein kann das erfindungsgemäße Filterelement so aufgebaut sein, dass ein Ausgangsmaterial mit den drei übereinander angeordneten Lagen, welche entsprechend der gewünschten Länge und einer doppelten Breite konfektioniert sind, einmal gefaltet und übereinandergeschlagen werden. Zum Bilden des insbesondere schlauchförmigen Filterelementes kann dann ein festes Verbinden entlang der Längskante erfolgen, so dass ein Filterelement mit nur einem Längsverbindungssaum gebildet ist. Die Schlauchform kann zylindrisch oder konisch sein.

Eine besonders stabile Ausgestaltung der Erfindung besteht darin, dass ein Aufbau aus zwei Hälften vorgesehen ist und dass die zwei Hälften entlang der beiden Längsränder unter Ausbildung von zwei Längsverbindungssäumen fest miteinander verbunden sind. Die jeweiligen Hälften können dabei gleich konfektioniert werden und weisen jeweils die drei Lagen auf. Dabei ist erfindungsgemäße die Zwischenlage steht definiert axial kürzer als die Außenlage und die Innenlage.

Eine weitere bevorzugte Ausführungsform der Erfindung besteht darin, dass eine axiale Länge des Überlaufbereiches zwischen 5% bis 30% der axialen Länge des Filterelementes beträgt. In dem eigentlichen Filterbereich ist eine Seitenwand des Filterelementes dreilagig ausgebildet, während in dem Überlaufbereich ein zweilagiger Aufbau aus der Außenlage und der Innenlage ohne Zwischenlage gebildet ist.

Grundsätzlich kann ein Verbinden der Lagen mit jedem geeigneten Verbindungsverfahren erfolgen. Fertigungstechnisch besonders vorteilhaft ist es, dass mindestens ein Verbindungssaum durch Ultraschallverschweißen gebildet ist. Dadurch lassen sich insbesondere Polymermaterialien mit einer geringen Verbindungsbreite effizient und zuverlässig bei einem schmalen und sauberen Verbindungssaum fest miteinander verbinden.

Zum Bilden eines taschenförmigen Filterelementes, insbesondere eines Blutfilterelementes, ist es nach einer Ausführungsvariante der Erfindung vorgesehen, dass der untere Querverbindungssaum zum Bilden eines geschlossenen Bodens ausgebildet ist. Hierdurch wird ein freier Durchtritt von Fluid, wie beispielsweise Blut, nach unten aus dem Filterelement heraus unterbunden. Es kann so eine Reinigung des gesamten Fluides sichergestellt werden, welches in das Filterelement eintritt.

Generell kann die feinporige Zwischenlage zum Filtern in jeder geeigneten Weise ausgebildet sein. Besonders zweckmäßig ist es nach einer Weiterbildung der Erfindung, dass das Filterelement ein Filtergewebe, eine Filtermembran, ein Filtergewirk und/oder ein Filtervlies aufweist. Entsprechend der vorgesehenen Filtrationsaufgabe sind eine Poren- oder Öffnungsgröße, ein Öffnungsanteil an der Filterfläche sowie eine Filterleistung oder ein Durchflusswiderstand auszuwählen.

Besonders vorteilhaft ist es nach einer Weiterbildung der Erfindung, dass eine Öffnungs- oder Porengröße des Filterelementes zwischen 10 µm und 400 µm liegt, insbesondere zwischen 25 µm und 60 µm. Dies kann insbesondere durch die Ausbildung des Filterelementes als ein Filtergewebe erreicht werden, wobei vorzugsweise ein Öffnungsanteil zwischen 30% bis 60% an der Gesamtfilterfläche erreicht werden kann. Dies ist insbesondere durch die Verwendung sehr feiner Fäden möglich, die vorzugsweise zwischen 10 µm bis 30 µm, besonders bevorzugte zwischen 18 µm bis 28 µm betragen.

Grundsätzlich weisen die Außenlage und die Innenlage eine durchlässige Struktur auf, insbesondere mit einer größeren Öffnungs- oder Porengröße wie die Zwischenlage. Nach einer Ausbildungsvariante der Erfindung ist es vorteilhaft, dass die Außenlage und/oder die Innenlage ausgebildet ist als ein Gewebe, ein Gewirk, Netz, Gitter und/oder Vlies. Besonders zweckmäßig ist der Einsatz eines Gewebes oder eines Gewirkes, welches vorzugsweise mit monofilen bzw. multifilen Fäden gebildet ist. Hierdurch wird ein Lösen von Teilen aus der Lage weitgehend unterbunden.

Eine besonders gute Reinigungswirkung wird dadurch erzielt, dass das Material der Zwischenlage, der Außenlage und der Innenlage ein Polymermaterial ist, insbesondere Polyester oder Polyamid. Bei dem Material handelt es sich um ein medizinisch zugelassenes, besonders reines Polymermaterial.

Nach einer Weiterbildung der Erfindung ist es zudem besonders zweckmäßig, dass das Material der Zwischenlage, der Außenlage und der Innenlage gleich ist. Die Verwendung des gleichen Materials verbessert die Anwendbarkeit im medizinischen Sektor und erleichtert auch eine Zulassung als medizinisches Produkt. Insbesondere in Kombination mit einem Verschweißen, insbesondere einem Ultraschallverschwei-ßen, kann ein sortenreines Filterelement gebildet werden, welches frei von zusätzlichen Klebstoffen oder anderen Materialien ist. Hierdurch kann eine besonders hohe Produktsicherheit gewährleistet werden.

Eine weitere vorteilhafte Ausführungsform der Erfindung besteht darin, dass eine Öffnungsgröße der Außenlage und/0der der Innenlage zwischen 100 µm und 400 µm beträgt, insbesondere zwischen 150 µm und 300 µm. Hierdurch wird ein Durchflusswiderstand in dem Filterelement in Bezug auf die maßgeblich filternde Zwischenlage kaum beeinträchtigt. Zudem wird mit diesem Größenbereich insbesondere bei einem Öffnungsanteil von über 50% in der Außenlage und der Innenlage, eine gute Überlauffunktion für den Überlaufbereich erreicht.

Bezüglich des Verfahrens zum Herstellen eines Filterelementes, insbesondere eines Blutfilterelementes, ist die Erfindung dadurch gekennzeichnet, dass die Außenlage und die Innenlage an ihren Oberkanten unter Ausbildung eines oberen Querverbindungssaumes fest miteinander verbunden werden, wobei die Zwischenlage in einem Zwischenraum zwischen der Außenlage und der Innenlage angeordnet ist und eine Oberkante der Zwischenlage frei und ungebunden bleibt. Hierdurch wird ein Filterelement mit einer Überlauffunktion in einem oberen Bereich durch eine nur Zweilagenaufbau erreicht, wobei eine Oberkante der filtrierenden Zwischenlage frei und ungebunden zwischen der Außenlage und der Innenlage verbleibt.

Durch das Verfahren kann insbesondere das zuvor beschriebene Filterelement, wie ein Blutfilterelement, effizient hergestellt werden, wobei die zuvor beschriebenen Vorteile erreicht werden.

Eine besonders gute Verbindung zwischen den einzelnen flexiblen Lagen kann nach einer Weiterbildung der Erfindung dadurch bereitgestellt werden, dass ein Verbinden der Außenlage, der Innenlage und/oder der Zwischenlage an ihren Außenrändern über ein Ultraschallverschweißen, ein thermisches Verschweißen, ein Kleben und/oder ein Heißkleben erfolgt. Insbesondere bei einem Ultraschallverschweißen und bei einem thermischen Verschweißen kann der Einsatz von Klebstoffen oder zusätzlichen Komponenten entfallen.

Nach einer weiteren Verfahrensvariante der Erfindung ist es zweckmäßig, dass durch ein Verbinden der flexiblen Lagen eine taschen- oder schlauchförmige Zwischenform mit außenliegenden Verbindungssäumen gebildet wird, und dass die Zwischenform zum Bilden des Filterelementes gestülpt wird, wobei die Verbindungssäume nach innen gedreht werden. Hierdurch kann eine geschützte Anordnung der Verbindungssäume am Zwischenprodukt erzielt werden. Bei einer Anströmung des Filterelementes von innen wird zudem sichergestellt, dass sich selbst bei einem Lösen kleinster Bestandteile aus dem Verbindungssaum diese bei der Verwendung als Blutfilterelement nicht in den eigentlichen Blutkreislauf gelangen können, da sie dann von der in Strömungsrichtung nachfolgenden filtrierenden Zwischenlage aufgefangen werden.

Wie bereits erwähnt kann das erfindungsgemäße Filterelement bevorzugt als Blutfilterelement beispielsweise in einer Herz-Lungen-Maschine verwendet werden. Es eignet sich aber ebenso zum Filtern von anderen Fluiden, unter denen nach der Erfindung insbesondere Flüssigkeiten mit oder ohne Feststoffen sowie mit oder ohne Gasanteilen verstanden werden können.

Die Erfindung wird nachfolgend anhand von bevorzugten Ausführungen weiter beschrieben, welche schematisch in den Zeichnungen dargestellt sind. In den Zeichnungen zeigen:
- Fig. 1: eine Seitenansicht eines ersten erfindungsgemäßen Filterlementes;
- Fig. 2: eine Querschnittsansicht des Filterelementes von Fig. 1;
- Fig. 3: eine Draufsicht von oben auf das Filterelement nach den Figuren 1 und 2;
- Fig. 4: eine vergrößerte Darstellung des Details A von Fig. 2;
- Fig. 5: eine vergrößerte Darstellung des Details B von Fig. 2;
- Fig. 6: eine Seitenansicht eines zweiten erfindungsgemäßen Filterelementes;
- Fig. 7: eine Querschnittsansicht des Filterelementes von Fig. 6;
- Fig. 8: eine Draufsicht von oben auf das Filterelement nach den Figuren 6 und 7;
- Fig. 9: eine vergrößerte Darstellung des Details A von Fig. 7; und
- Fig. 10: eine vergrößerte Darstellung des Details B von Fig. 7;
- Fig. 11: eine Seitenansicht eines dritten erfindungsgemäßen Filterelementes;
- Fig. 12: eine Querschnittsansicht des Filterelementes von Fig. 11;
- Fig. 13: eine Draufsicht von oben auf das Filterelement nach den Figuren 11 und 12;
- Fig. 14: eine vergrößerte Darstellung des Details A von Fig. 12; und
- Fig. 15: eine vergrößerte Darstellung des Details B von Fig. 12
- Fig. 16: eine Seitenansicht eines vierten erfindungsgemäßen Filterelementes;
- Fig. 17: eine Querschnittsansicht des Filterelementes von Fig. 16;
- Fig. 18: eine Draufsicht von oben auf das Filterelement nach den Figuren 16 und 17;
- Fig. 19: eine vergrößerte Darstellung des Details A von Fig. 17; und
- Fig. 20: eine vergrößerte Darstellung des Details B von Fig. 17.

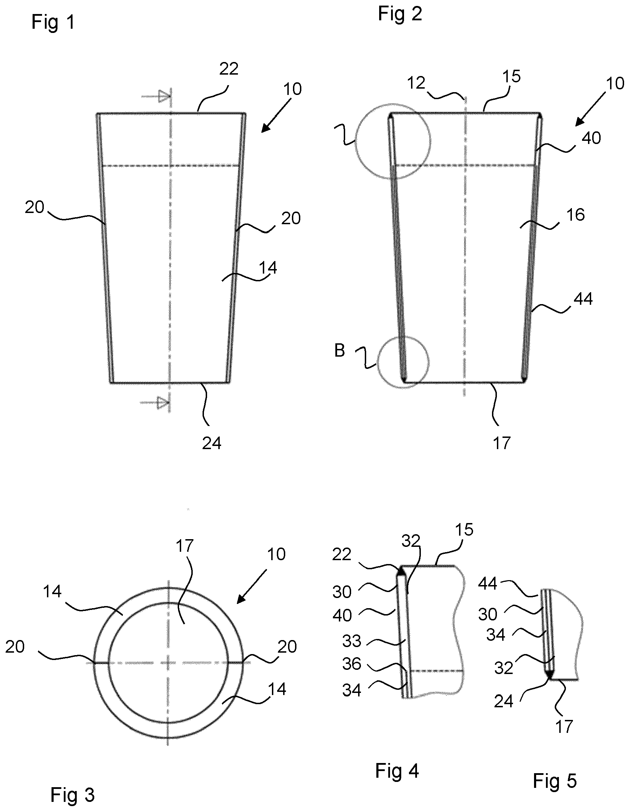

Gemäß den Figuren 1 bis 5 ist ein erstes Ausführungsbeispiel eines erfindungsgemäßen Filterelementes 10 dargestellt, welches schlauchförmig mit einem durchgehenden Innenraum 16 mit einer oberen Öffnung 15 und einer unteren Öffnung 17 ausgebildet ist. Das schlauchförmige Filterelement 10 ist leicht konisch entlang einer Längsachse 12 zur unteren Öffnung 17 hin. Die Öffnungen 15, 17 sind etwa kreisförmig ausgebildet.

Das Filterelement 10 ist aus zwei Hälften 14 gebildet, welche entlang von zwei Längsrändern entlang der Längsachse 12 mit Längsverbindungssäumen 20 miteinander verbunden sind. Das Verbinden kann insbesondere mittels eines Ultraschallverscheißens erfolgen. Jede Hälfte 14 weist drei Lagen auf, nämlich eine Außenlage 30, eine Innenlage 32 und eine dazwischen in einem Zwischenraum 33 angeordnete Zwischenlage 34. Die Zwischenlage 34 stellt ein feinporiges Filterelement dar, während die Außenlage 30 und die Innenlage 32 größere Porenöffnungen aufweisen können. Die Außenlage 30 und die Innenlage 32 weisen dabei die gleiche axiale Länge auf, während die Zwischenlage 34 definiert axial kürzer ausgebildet ist.

Bei dem dargestellten Filterelement 10 sind die drei Lagen entlang der zwei Längsverbindungssäume 20 sowie entlang eines unteren Querverbindungssaumes 24 etwa durch Ultraschallverschweißen miteinander verbunden. Der untere Querverbindungssaum 24 ist ringförmig ausgebildet und umschließt so die untere Öffnung 17. Der Bereich mit den insgesamt drei Lagen bildet einen Filterbereich 44, welcher sich etwa über 80% der axialen Länge des Filterelementes 10 von unten nach oben erstreckt.

In einem oberen Bereich ist eine Wandung des Filterelementes 10 lediglich zweilagig mit der Außenlage 30 und der Innenlage 32 ausgebildet, wie anschaulich in Fig. 4 dargestellt ist. Die Außenlage 30 und die Innenlage 32 sind über einen ringförmigen oberen Querverbindungssaum 22 miteinander verschweißt, wobei der Zwischenraum 33 abgeschlossen ist. Dieser zweilagige Abschnitt bildet einen Überlaufbereich 40 in dem Filterelement 10, welcher bei einem Fluidrückstau nach oben aufgrund des deutlich geringeren Durchflusswiderstandes ein verbessertes Fließen des Fluides durch die Wandung hindurch ermöglicht.

An dem unteren Ende des Überlaufbereiches 40 endet die Zwischenlage 34, wobei eine Oberkante 36 der Zwischenlage 34 frei in dem Zwischenraum 33 zwischen der Außenlage 30 und der Innenlage 32 zu liegen kommt, ohne dass eine direkte fest Verbindung der Oberkante 36 mit der angrenzenden Außenlage 30 oder der angrenzenden Innenlage 32 gegeben ist.

Gemäß Fig. 5 sind zum Abschluss des unteren Filterbereiches 44 die Außenlage 30, die Innenlage 32 und die Zwischenlage 34 über den ringförmigen unteren Querverbindungssaum 24 miteinander verschweißt und fest verbunden.

Bei dem dargestellten Ausführungsbeispiel erfolgt eine Anströmung des zu reinigenden Fluides durch die obere Öffnung 15 entlang der Längsachse 12. Durch eine anliegende Druckdifferenz kann das Fluid aus einem Innenraum 16 des Filterelementes 10 radial nach außen strömen. Bei einem normalen Füllungsverhältnis erfolgt dies durch den dreilagigen Filterbereich 44, während bei einem eventuellen Rückstau eine radiale Durchströmung auch in dem oberen Überlaufbereich 40 erfolgen kann. Bei der Verwendung als Blutfilterelement kann die Porenstruktur der Außenlage 30 und der Innenlage 32 sind dabei so ausgebildet sein, dass größere und für einen Patienten kritische Partikelgrößen und Gasbläschen rückgehalten werden, auch wenn nicht die gleiche Filter- und Reinigungswirkung wie bei einer Durchströmung in dem Filterbereich 44 mit der zusätzlichen Zwischenlage 34 erzielt wird.

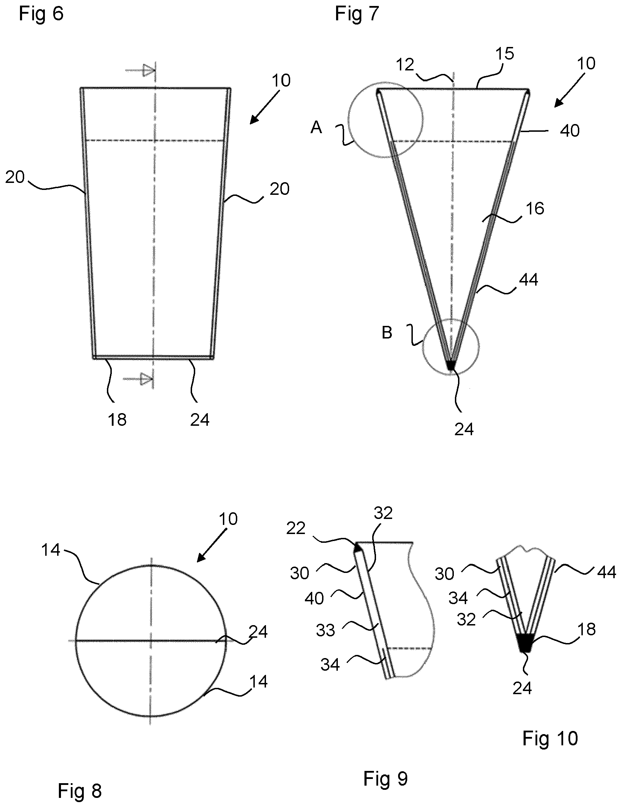

Eine weitere Ausgestaltungsmöglichkeit eines erfindungsgemäßen Filterelementes 10 ist in den Figuren 6 bis 10 dargestellt. Dieses Filterelement 10 weist den grundsätzlich gleichen Aufbau wie das zuvor beschriebene Filterelement 10 nach den Figuren 1 bis 5 auf, wobei jedoch eine taschenartige Struktur mit einem geschlossenen Boden 18 gebildet ist. Das Filterelement 10 ist dabei ebenfalls aus zwei Hälften 14 durch Übereinanderlegen und Verschweißen entlang der Längsränder mit Längsverbindungssäumen 20 hergestellt. Zusätzlich sind die Außenlage 30, die Innenlage 32 und die dazwischen angeordnete Zwischenlage 34 auch an ihren unteren Enden über einen linienförmigen unteren Querverbindungssaum 24 fest miteinander verbunden, wobei ein geschlossener Boden 18 gebildet ist.

Die Zwischenlage 34, welche das eigentliche feinporige Filterelement umfasst, erstreckt sich dabei vom unteren Ende bis zu etwa 80% der Gesamtlänge des Filterelementes 10, wobei ein dreilagiger Filterbereich 44 gebildet ist. In dem restlichen zweilagigen oberen Bereich mit der Außenlage 30 und der Innenlage 32 ist ein Überlaufbereich 40 gebildet, welcher eine leichtere Durchströmung der Wandung des Filterelementes 10 ermöglicht.

Auch bei diesem Ausführungsbeispiel eines Filterelementes 10 erfolgt ein Anströmen des zu reinigenden Fluides von oben über eine im Wesentlichen kreisförmige Öffnung 15 in einen Innenraum 16 des Filterelementes 10. Aufgrund einer anliegenden Druckdifferenz kann das Fluid von innen nach außen die Wandung entweder des dreilagigen Filterbereiches 44 oder bei einer entsprechenden Füllhöhe durch den zweilagigen Überlaufbereich 40 radial nach außen erfolgen.

Am oberen Ende sind die Außenlage 30 und die Innenlage 32 unmittelbar über einen oberen Querverbindungssaum 22 miteinander verschweißt, welcher zum Bilden der oberen Öffnung ringförmig ausgebildet ist. Insgesamt wird so ein sockenartiger oder zeltförmiger Aufbau des Filterelementes 10 erreicht.

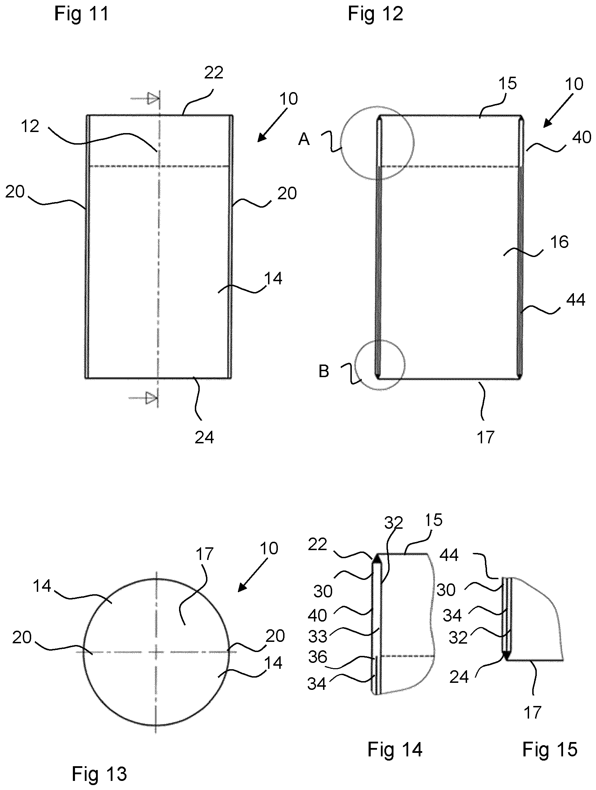

Eine dritte Ausführungsform eines erfindungsgemäßen Filterelementes 10 ist in den Figuren 11 bis 15 dargestellt. Dieses Filterelement 10 gleich im Wesentlichem dem Filterelement 10 gemäß der ersten Ausführungsform, welche in den Figuren 1 bis 5 gezeigt ist. Es unterscheidet sich lediglich zur dieser ersten Ausführungsform dadurch, dass wie durch den Vergleich der Figuren 1 und 2 mit den Figuren 11 und 12 ersichtlich, sich das Filterelement 10 nach unten nicht verjüngt. Es hat demnach eine im Wesentlichen zylinderähnliche Form.

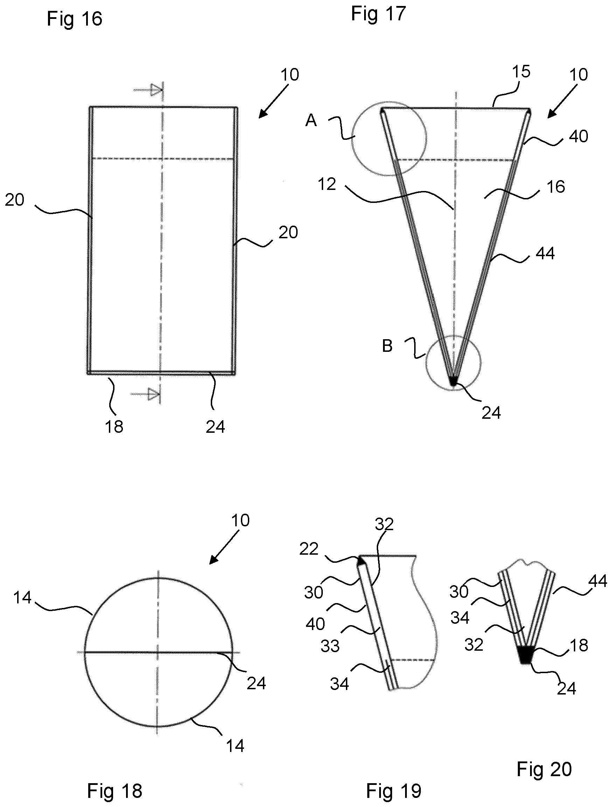

Eine vierte Ausführungsform eines erfindungsgemäßen Filterelementes 10 ist in den Figuren 16 bis 20 dargestellt. Diese Filterelement 10 entspricht im Wesentlichen der zweiten Ausführungsform, wobei es ebenfalls eine taschenartige Struktur mit einem geschlossenen Boden 18 aufweist. Im Gegensatz zum zweiten Ausführungsbeispiel verläuft diese Ausführungsform allerdings nur in einer Dimension konisch zur untern Öffnung 17 hin. Dies wird insbesondere durch den Vergleich der Figuren 6 und 16 sichtbar.

## Patentansprüche

1. Filterelement, insbesondere Blutfilterelement, welches taschen- oder schlauchförmig mit einem sich in einer Längsrichtung erstreckenden Innenraum (16) ausgebildet ist und einen mehrlagigen Aufbau aufweist, welcher eine durchlässige Außenlage (30), eine durchlässige Innenlage (32) und mindestens eine zwischen der Außenlage (30) und der Innenlage (32) angeordnete Zwischenlage (34) aufweist, welche als ein feinporiges Filterelement ausgebildet ist, wobei das Filterelement (10) eine vorgegebene axiale Länge aufweist und die Außenlage (30) und die Innenlage (32) die vorgegebene axiale Länge aufweisen,
**dadurch gekennzeichnet,**
**dass** die Zwischenlage (34) axial kürzer als die Außenlage (30) und die Innenlage (32) unter Ausbildung eines Überlaufbereiches (40) ausgebildet ist, welcher an einem axialen oberen Endbereich des Filterelementes (10) angeordnet ist, wobei eine Oberkante (36) der Zwischenlage (34) von der Oberkante der Außenlage (30) und der Innenlage (32) axial nach unten beabstandet ist,
**dass** die Außenlage (30), die Innenlage (32) und die Zwischenlage (34) entlang mindestens einem Längsrand unter Ausbildung eines Längsverbindungssaumes (20) fest miteinander verbunden sind und
**dass** die Außenlage (30) und die Innenlage (32) entlang ihrer Oberkante unter Ausbildung eines oberen Querverbindungssaumes (22) fest miteinander verbunden sind, wobei die Oberkante der Zwischenlage (34) frei und unverbunden zwischen der Außenlage (30) und der Innenlage (32) angeordnet ist.

2. Filterelement nach Anspruch 1,
**dadurch gekennzeichnet,**
**dass** die Außenlage (30), die Innenlage (32) und/oder die Zwischenlage (34) an ihrer Unterkante unter Ausbildung eines unteren Querverbindungssaumes (24) miteinander fest verbunden sind.

3. Filterelement nach Anspruch 1 oder 2,
**dadurch gekennzeichnet,**
**dass** ein Aufbau aus zwei Hälften (14) vorgesehen ist und
**dass** die zwei Hälften (14) entlang der beiden Längsränder unter Ausbildung von zwei Längsverbindungssäumen (20) fest miteinander verbunden sind.

4. Filterelement nach einem der Ansprüche 1 bis 3,
**dadurch gekennzeichnet,**
**dass** eine axiale Länge des Überlaufbereiches (40) zwischen 5% bis 30% der axialen Länge des Filterelementes (10) beträgt.

5. Filterelement nach einem der Ansprüche 1 bis 4,
**dadurch gekennzeichnet,**
**dass** mindestens ein Verbindungssaum (20, 22, 24) durch Ultraschallverschwei-ßen gebildet ist.

6. Filterelement nach einem der Ansprüche 2 bis 5,
**dadurch gekennzeichnet,**
**dass** der untere Querverbindungssaum (24) zum Bilden eines geschlossenen Bodens (18) ausgebildet ist.

7. Filterelement nach einem der Ansprüche 1 bis 6,
**dadurch gekennzeichnet,**
**dass** das Filterelement ein Filtergewebe, eine Filtermembran, ein Filtergewirk und/oder ein Filtervlies aufweist.

8. Filterelement nach einem der Ansprüche 1 bis 7,
**dadurch gekennzeichnet,**
**dass** eine Öffnungs- oder Porengröße des Filterelementes zwischen 10 µm und 400 µm liegt, insbesondere zwischen 25 und 60 µm.

9. Filterelement nach einem der Ansprüche 1 bis 8,
**dadurch gekennzeichnet,**
**dass** die Außenlage (30) und/oder die Innenlage (32) ausgebildet ist als ein Gewebe, Gewirk, Netz, Gitter und/oder Vlies.

10. Filterelement nach einem der Ansprüche 1 bis 9,
**dadurch gekennzeichnet,**
**dass** das Material der Zwischenlage (34), der Außenlage (30) und der Innenlage (32) ein Polymermaterial ist, insbesondere Polyester oder Polyamid.

11. Filterelement nach einem der Ansprüche 1 bis 10,
**dadurch gekennzeichnet,**
**dass** das Material der Zwischenlage (34), der Außenlage (30) und der Innenlage (34) gleich ist.

12. Filterelement nach einem der Ansprüche 1 bis 11,
**dadurch gekennzeichnet,**
**dass** eine Öffnungsgröße der Außenlage (30) und/oder der Innenlage (32) zwischen 100 µm und 400 µm beträgt, insbesondere zwischen 150 µm und 300 µm.

13. Verfahren zum Herstellen eines Filterelement (10), insbesondere eines Blutfilterelementes, nach einem der Ansprüche 1 bis 12,
**dadurch gekennzeichnet,**
**dass** die Außenlage und die Innenlage (32) an ihren Oberkanten unter Ausbildung eines oberen Querverbindungssaumes (22) fest miteinander verbunden werden, wobei die Zwischenlage (34) in einem Zwischenraum (33) zwischen der Außenlage (30) und der Innenlage (32) angeordnet ist und eine Oberkante der Zwischenlage (34) frei und ungebunden bleibt.

14. Verfahren nach Anspruch 13,
**dadurch gekennzeichnet,**
**dass** ein Verbinden der Außenlage (30), der Innenlage (32) und/oder der Zwischenlage (34) an ihren Außenrändern über ein Ultraschallverschweißen, ein thermisches Verschweißen, ein Kleben und/oder ein Heißverkleben erfolgt.

15. Verfahren nach Anspruch 13 oder 14,
**dadurch gekennzeichnet,**
**dass** durch ein Verbinden der Lagen (30, 32, 34) eine taschen- oder schlauchförmige Zwischenform mit außenliegenden Verbindungssäumen (20, 22, 24) gebildet wird und
**dass** die Zwischenform zum Bilden des Filterelementes (10) gestülpt wird, wobei die Verbindungssäume (20, 22, 24) nach innen gedreht werden.

## Claims

1. Filter element, in particular blood filter element, which is configured in the form of a pocket or tube with an inner space (16) extending in a longitudinal direction and has a multilayer structure which comprises a permeable outer layer (30), a permeable inner layer (32) and at least one intermediate layer (34) which is arranged between the outer layer (30) and the inner layer (32) and is configured as a fine-pored filter element, wherein the filter element (10) has a predetermined axial length and the outer layer (30) and the inner layer (32) have the predetermined axial length,
**characterized in that**
the intermediate layer (34) is formed axially shorter than the outer layer (30) and the inner layer (32) along with the formation of an overflow region (40) which is arranged at an axial upper end area of the filter element (10), wherein an upper edge (36) of the intermediate layer (34) is axially spaced downwards from the upper edge of the outer layer (30) and the inner layer (32),
**in that** the outer layer (30), the inner layer (32) and the intermediate layer (34) are firmly connected to one another along at least one longitudinal edge along with the formation of a longitudinal connecting seam (20), and
**in that** the outer layer (30) and the inner layer (32) are firmly connected to one another along their upper edge along with the formation of an upper transverse connecting seam (22), wherein the upper edge of the intermediate layer (34) is arranged free and unconnected between the outer layer (30) and the inner layer (32).

2. Filter element according to claim 1,
**characterized in that**
the outer layer (30), the inner layer (32) and/or the intermediate layer (34) are firmly connected to one another at their lower edge along with the formation of a lower transverse connecting seam (24).

3. Filter element according to claim 1 or 2,
**characterized in that**
a structure composed of two halves (14) is provided and
**in that** the two halves (14) are firmly connected to one another along the two longitudinal edges along with the formation of two longitudinal connecting seams (20).

4. Filter element according to any one of claims 1 to 3,
**characterized in that**
an axial length of the overflow region (40) is between 5% to 30% of the axial length of the filter element (10).

5. Filter element according to any one of claims 1 to 4,
**characterized in that**
at least one connecting seam (20, 22, 24) is formed by ultrasonic welding.

6. Filter element according to any one of claims 2 to 5,
**characterized in that**
the lower transverse connecting seam (24) is configured to form a closed bottom (18).

7. Filter element according to any one of claims 1 to 6,
**characterized in that**
the filter element comprises a filter woven fabric, a filter membrane, a filter knitted fabric and/or a non-woven filter fabric.

8. Filter element according to any one of claims 1 to 7,
**characterized in that**
an aperture or pore size of the filter element is between 10 µm and 400 µm, in particular between 25 and 60 µm.

9. Filter element according to any one of claims 1 to 8,
**characterized in that**
the outer layer (30) and/or the inner layer (32) is formed as a woven fabric, knitted fabric, net, grate and/or nonwoven fabric.

10. Filter element according to any one of claims 1 to 9,
**characterized in that**
the material of the intermediate layer (34), the outer layer (30) and the inner layer (32) is a polymeric material, in particular polyester or polyamide.

11. Filter element according to any one of claims 1 to 10,
**characterized in that**
the material of the intermediate layer (34), the outer layer (30) and the inner layer (34) is the same.

12. Filter element according to any one of claims 1 to 11,
**characterized in that**
an aperture size of the outer layer (30) and/or the inner layer (32) is between 100 µm and 400 µm, in particular between 150 µm and 300 µm.

13. Method for producing a filter element (10), in particular a blood filter element, according to any one of claims 1 to 12,
**characterized in that**
the outer layer and the inner layer (32) are firmly connected to one another at their upper edges along with the formation of an upper transverse connecting seam (22), wherein the intermediate layer (34) is arranged in an intermediate space (33) between the outer layer (30) and the inner layer (32), and an upper edge of the intermediate layer (34) remains free and unattached.

14. Method according to claim 13,
**characterized in that**
the outer layer (30), the inner layer (32) and/or the intermediate layer (34) are connected at their outer edges by ultrasonic welding, thermal welding, adhesive bonding and/or hot bonding.

15. Method according to claim 13 or 14,
**characterized in that**
by connecting the layers (30, 32, 34) a pocket- or tube-shaped intermediate shape with external connecting seams (20, 22, 24) is formed, and
**in that** the intermediate shape is turned inside out to form the filter element (10), wherein the connecting seams (20, 22, 24) are turned inwards.

## Revendications

1. Elément filtrant, en particulier élément filtrant pour le sang, qui est conçu sous la forme d'une poche ou d'un tuyau avec un espace interne (16) qui s'étend dans une direction longitudinale et présente une structure multicouche qui présente une couche externe perméable (30), une couche interne perméable (32) et au moins une couche intermédiaire (34) disposée entre la couche externe (30) et la couche interne (32), qui est conçue comme un élément filtrant à pores fins, où l'élément filtrant (10) présente une longueur axiale prédéterminée et la couche externe (30) et la couche interne (32) présentent la longueur axiale prédéterminée,
**caractérisé**
**en ce que** la couche intermédiaire (34) est conçue axialement plus courte que la couche externe (30) et la couche interne (32) avec formation d'une zone de débordement (40) qui est disposée au niveau d'une zone d'extrémité supérieure axiale de l'élément filtrant (10), où une arête supérieure (36) de la couche intermédiaire (34) est espacée axialement vers le bas de l'arête supérieure de la couche externe (30) et de la couche interne (32),
**en ce que** la couche externe (30), la couche interne (32) et la couche intermédiaire (34) sont liées solidement entre elles le long d'au moins un bord longitudinal avec formation d'une couture de liaison longitudinale (20) et
**en ce que** la couche externe (30) et la couche interne (32) sont liées solidement entre elles le long de leur arête supérieure avec formation d'une couture de liaison transversale supérieure (22), où l'arête supérieure de la couche intermédiaire (34) est disposée libre et non liée entre la couche externe (30) et la couche interne (32).

2. Elément filtrant selon la revendication 1,
**caractérisé**
**en ce que** la couche externe (30), la couche interne (32) et/ou la couche intermédiaire (34) sont liées solidement entre elles au niveau de leur arête inférieure avec formation d'une couture de liaison transversale inférieure (24).

3. Elément filtrant selon la revendication 1 ou 2,
**caractérisé**
**en ce qu'**il est prévu une structure composée de deux moitiés (14) et
**en ce que** les deux moitiés (14) sont liées solidement entre elles le long des deux bords longitudinaux avec formation de deux coutures de liaison longitudinales (20).

4. Elément filtrant selon l'une des revendications 1 à 3,
**caractérisé**
**en ce qu'**une longueur axiale de la zone de débordement (40) représente entre 5 % et 30 % de la longueur axiale de l'élément filtrant (10).

5. Elément filtrant selon l'une des revendications 1 à 4,
**caractérisé**
**en ce qu'**au moins une couture de liaison (20, 22, 24) est formée par soudage par ultrasons.

6. Elément filtrant selon l'une des revendications 2 à 5,
**caractérisé**
**en ce que** la couture de liaison transversale inférieure (24) est conçue pour former un fond fermé (18).

7. Elément filtrant selon l'une des revendications 1 à 6,
**caractérisé**
**en ce que** l'élément filtrant présente un tissu filtrant, une membrane filtrante, un tricot filtrant et/ou un non-tissé filtrant.

8. Elément filtrant selon l'une des revendications 1 à 7,
**caractérisé**
**en ce qu'**une taille d'ouverture ou de pores de l'élément filtrant est située entre 10 µm et 400 µm, en particulier entre 25 et 60 µm.

9. Elément filtrant selon l'une des revendications 1 à 8,
**caractérisé**
**en ce que** la couche externe (30) et/ou la couche interne (32) sont conçues sous forme d'un tissu, d'un tricot, d'un filet, d'une grille et/ou d'un non-tissé.

10. Elément filtrant selon l'une des revendications 1 à 9,
**caractérisé**
**en ce que** le matériau de la couche intermédiaire (34), de la couche externe (30) et de la couche interne (32) est un matériau polymère, en particulier polyester ou polyamide.

11. Elément filtrant selon l'une des revendications 1 à 10,
**caractérisé**
**en ce que** le matériau de la couche intermédiaire (34), de la couche externe (30) et de la couche interne (34) est le même.

12. Elément filtrant selon l'une des revendications 1 à 11,
**caractérisé**
**en ce qu'**une taille d'ouverture de la couche externe (30) et/ou de la couche interne (32) est entre 100 µm et 400 µm, en particulier entre 150 µm et 300 µm.

13. Procédé de fabrication d'un élément filtrant (10), en particulier d'un élément filtrant pour le sang, selon l'une des revendications 1 à 12,
**caractérisé**
**en ce que** la couche externe et la couche interne (32) sont liées solidement entre elles au niveau de leurs arêtes supérieures avec formation d'une couture de liaison transversale supérieure (22), où la couche intermédiaire (34) est disposée dans un espace intermédiaire (33) entre la couche externe (30) et la couche interne (32) et une arête supérieure de la couche intermédiaire (34) reste libre et non liée.

14. Procédé selon la revendication 13,
**caractérisé**
**en ce qu'**une liaison de la couche externe (30), de la couche interne (32) et/ou de la couche intermédiaire (34) a lieu au niveau de leurs bords externes par un soudage par ultrasons, un soudage thermique, un collage et/ou un thermocollage.

15. Procédé selon la revendication 13 ou 14,
**caractérisé**
**en ce que** par une liaison des couches (30, 32, 34), une forme intermédiaire en forme de poche ou de tuyau avec des coutures de liaison (20, 22, 24) situées à l'extérieur est formée et
**en ce que** la forme intermédiaire est retournée pour former l'élément filtrant (10), où les coutures de liaison (20, 22, 24) sont tournées vers l'intérieur.
